# NEUE EUROPÄISCHE PATENTSCHRIFT

(11) **EP 3 359 229 B2**
(45) Veröffentlichungstag und Bekanntmachung des Hinweises auf die Entscheidung über den Einspruch: **12.03.2025**
(45) Hinweis auf die Patenterteilung: 18.05.2022
(21) Anmeldenummer: 16757813.7
(22) Anmeldetag: 18.08.2016
(51) Int. Cl.: A61M 5/32, A61M 5/50, A61M 5/20, G06K 19/077, G09F 3/03

(54) **INJEKTOR MIT ZERSTÖRBAREM ELEKTRISCHEM SCHALTKREIS INFOLGE BENUTZUNG**
INJECTOR WITH DESTROYABLE ELECTRIC CIRCUIT DUE TO USAGE
INJECTEUR AVEC CIRCUIT ELECTRIQUE DESTRUCTIBLE PAR SUITE D'USAGE

(30) Priorität: 07.10.2015 CH 14582015
(43) Veröffentlichungstag der Anmeldung: 15.08.2018
(73) Patentinhaber: Ypsomed AG, 3401 Burgdorf (CH)
(72) Erfinder: SCHNEIDER, Andreas, 3027 Bern (CH); URBANEK, Leos, 3012 Bern (CH)
(74) Vertreter: Meier Obertüfer, Jürg
(86) Internationale Anmeldenummer: PCT/CH2016/000109
(87) Internationale Veröffentlichungsnummer: WO 2017/059553

(56) Entgegenhaltungen:
- WO-A1-2014/183226
- DE-A1- 102012 112 297
- US-A- 3 126 004
- US-A1- 2006 152 364
- US-A1- 2007 095 160
- US-A1- 2012 280 815

## Beschreibung

### Gebiet

Die Erfindung bezieht sich allgemein auf Geräte und Verfahren für die parenterale Verabreichung von Medikamenten. Beispielsweise Verabreichungsvorrichtungen wie Insulinpumpen, Patchpumpen, Bolusinjektoren, automatische Injektoren, Peninjektoren, Autopen aber auch herkömmliche Spritzen und vorgefüllte Spritzen. Im klinischen Umfeld und insbesondere in der Selbstmedikation sind klar definierte Rück- und Zustandsmeldungen an Patienten oder Pflegepersonal vorteilhaft für die Gewährleistung einer sicheren und effektiven Anwendung. Bei heute im Markt verfügbaren Verabreichungsgeräten schliesst der Patient aufgrund von visuellen, akustischen, haptischen oder taktilen Signalen auf den Benutzungszustand des Gerätes. Visuelle Signale können beispielsweise aufgrund ihrer Farb- oder Formänderung, akustisch mit Klickgeräusch, haptische oder taktile mit tastbaren Formänderungen oder aufgrund von mechanischen Beschleunigungen erkennbar sein. Oftmals sind solche Signale aber transient und können übersehen, überhört oder vergessen werden. Zusätzliche und neuartige Rückmeldungen und Bestätigungen insbesondere über den Benutzungszustand und den erfolgreichen Ablauf einer Verabreichung können massgeblich zur Verbesserung der Patientensicherheit, des Patientenvertrauens und der Therapietreue beitragen. Weiter können solche Rückmeldungen und Zustandsmeldungen bevorzugt auch aufgezeichnet und unmittelbar oder zeitverschoben ausgewertet oder mit Vorgaben verglichen werden. Weiter verbessert die Erfindung die Gewährleistung der Therapie durch den Leistungserbringer und erlaubt dem Leistungsträger eine Kontrolle auch wirtschaftlicher Aspekte.

### Hintergrund

Die PCT-Anmeldung mit internationaler Veröffentlichungsnummer WO2006/102678 A1 zeigt eine Erfindung welche Sicherheitsverpackungen "tamper-evident" mittels RFID-Tag bereitstellt. Die Erfindung betrifft Behälter und beansprucht das sichere Erkennen einer vorgängig erfolgten ersten Öffnung der Behälter. Das Erfassen und Signalisieren von spezifischen Benutzungszuständen einer Verabreichungsvorrichtung für Medikamente wird nicht gelehrt.

Die PCT-Anmeldung mit internationaler Veröffentlichungsnummer WO2009/140782 A1 zeigt eine Erfindung welche Kartuschen mit einer Druckmesseinrichtung und einer Übertragungseinrichtung bereitstellt. Die Erfindung lehrt und beansprucht Kartuschen mit Drucküberwachungseinrichtungen für die kontinuierliche Verabreichung von Flüssigkeiten. Das Erfassen und Signalisieren von spezifischen Benutzungszuständen von Verabreichungsvorrichtungen für Medikamente wird nicht gelehrt.

Die Erfindung aus der PCT-Anmeldung mit internationaler Veröffentlichungsnummer WO2013160152 A1 betrifft einen Einsatz für ein Medikament welcher eine Datenspeichervorrichtung und eine Spritze enthält. Ein integrales Trägermittel wird nicht gelehrt oder offenbart, welches diese Datenspeichervorichtung zusammen mit der Antenne und dem Sensor aufnimmt und verbindet.

Die Erfindung aus der PCT-Anmeldung mit internationaler Veröffentlichungsnummer WO2015071354 A1 betrifft eine Verabreichungsvorrichtung welche autonom einen Zeitparameter bereitstellt und zur Anzeige bringen kann. Dazu nötig ist dauernd eine Energiequelle, welche zusammen mit der offenbarten Zeitanzeige auch beansprucht wird. Eine passive Vorrichtung und entsprechende Verfahren zum Erfassen und Signalisieren von spezifischen Benutzungszuständen einer Verabreichungsvorrichtung für Medikamente ohne dauernde Energieversorgung wird nicht gelehrt. Die Patentanmeldung US 2006/0152364 betrifft RFID Einrichtungen zur Erkennung von Veränderungen oder Zuständen an einem Behälter für medizinische Pillen sowie Verbesserungen gegenüber der oft unzuverlässigen und starren Kennzeichnung von Fluggepäck mittels Barcodes.

### Aufgabe und Zusammenfassung

Es ist eine Aufgabe der vorliegenden Erfindung Vorrichtungen und Verfahren bereitzustellen, welche das Erfassen und Signalisieren von unterschiedlichen Benutzungszuständen einer Verabreichungsvorrichtung spezifischer oder einfacher oder sichererer erfüllen als die Lehren im Stand der Technik. Es ist eine Aufgabe der vorliegenden Erfindung eine gewünschte Therapie zu verbessern und wirtschaftlicher zu machen. Es ist eine Aufgabe der vorliegenden Erfindung, den erfolgreichen Ablauf einer parenteralen Verabreichung eines Medikaments sicher zu bestätigen.

Die Erfindung kann nach einem ihrer Aspekte wie folgt beschrieben werden: Eine Verabreichungsvorrichtung ist mit einem aktivierbaren Kennzeichnungsmittel versehen. Bevorzugt in Form eines bedruckbaren Mittels, welches eine elektronische Schaltung enthält wie sie beispielsweise als RFID Schaltung bekannt ist. Die elektronische Schaltung wird von einer Auslesevorrichtung drahtlos über eine Antennenstruktur temporär mit Energie versorgt und ist dadurch in der Lage Information zum aktuellen Benutzungszustand der Verabreichungsvorrichtung an die Auslesevorrichtung zu übermitteln. Erfindungsgemässe Ausführungsformen und Verfahren ergeben sich aus den unabhängigen Ansprüchen, vorteilhafte Weiterbildungen und Anwendungsfälle der Erfindung aus den abhängigen Ansprüchen, der Beschreibung und den Figuren.

### Allgemeine Beschreibung

Die Verabreichungsvorrichtungen. Die Erfindung geht von einer Verabreichungsvorrichtung zur parenteralen Verabreichung eines Produkts, insbesondere eines Medikaments aus. Beispielsweise kann die Verabreichungsvorrichtung ein Autoinjektor oder Bolusinjektor sein, welcher insbesondere folgende Mittel, welche aus einem oder mehreren Geräteteilen gebildet sind, enthalten kann:
- ein Gehäuse, welches vorzugsweise hülsenförmig und länglich mit einer Längsachse gebildet ist und einen vom distalen Ende abziehbaren Schutzdeckel aufweist
- einen Produktbehälter, insbesondere eine Spritze, an dessen distalem Ende eine Nadel oder Kanüle, insbesondere lösbar oder unlösbar angeordnet ist, in dem ein Kolben, der vorzugsweise dichtend an der ihn umgebenden Wand des Produktbehälters verschiebbar angeordnet ist und der in einem Produktbehälterhalter, der auch als Spritzenhalter bezeichnet werden kann, gehalten ist, wobei der Produktbehälterhalter axialfest mit dem Gehäuse vorzugsweise permanent verbunden, insbesondere verschnappt oder formschlüssig verbunden ist oder bezüglich des Gehäuses mittels der Kraft eins Antriebsmittels, insbesondere einer Druck- oder Triebfeder in distaler Richtung verschoben werden kann, wobei die Nadel aus dem Gehäuse über das distale Ende des Gehäuses ragt oder hervorsteht bzw. bewegt wird und vor der Verwendung mit einer Nadelhülle abgedeckt ist.
- eine Nadelschutzhülse, die insbesondere die Funktion einer Nadelschutzhülse und einer Auslöseeinrichtung für die Produktausschüttung inne hat, wobei die Nadelschutzhülse aus einer Ausgangsposition, in der das distale Ende der Nadelschutzhülse distal über die Nadelspitze der Nadel steht, so dass insbesondere der Zugriff auf die Nadel verhindert wird, insbesondere gegen die Kraft einer Nadelschutzhülsenfeder in das Gehäuse verschiebbar ist, insbesondere in die proximale Richtung, so dass die Nadel aus dem distalen Ende der Nadelschutzhülse hervortritt bzw. bewegt wird, insbesondere mit einer Länge hervortritt, die in etwa der Einstechtiefe der Nadel, vorzugsweise für eine subkutane Injektion, entspricht.
- einen betätigbaren Knopf welcher insbesondere zusammen mit der Nadelschutzhülse auf die Auslöseeinrichtung wirkt
- einen Stössel und eine Ausschüttfeder, wobei bevorzugt ist, dass die Ausschüttfeder eine als Druckfeder wirkende Wendelfeder ist, wobei der Stössel in dem Gehäuse angeordnet ist und von der insbesondere im Auslieferungszustand des Autoinjektors vorgespannten Ausschüttfeder, welche vorzugsweise zumindest teilweise innerhalb des vorzugsweise hülsenförmigen Stössels angeordnet ist, entlang einer Längsachse des Autoinjektors oder des Gehäuses in die distale Richtung verschiebbar ist, wenn der Stössel durch die Auslöseeinrichtung freigegeben wird, wobei die Verschiebung des Stössels mittels der Kraft der Ausschüttfeder in die distale Richtung bewirkt, dass der Kolben, an dem der Stössel zumindest bei der Verschiebung anstösst, von dem Stössel mitgenommen wird und das Produkt aus dem Produktbehälter, insbesondere über die Nadel, verdrängt.
- eine Sperreinrichtung, welche verhindert, dass die Nadelschutzhülse nach einer ersten Betätigung oder erfolgter Verabreichung erneut die abgedeckte Nadel freigeben kann.

Der Verabreichungsvorrichtung kann ferner ein Dosiseinstellelement umfassen, welches vorzugsweise eine Aussenfläche der Verabreichungsvorrichtung bildet und von dem Verwender der Verabreichungsvorrichtung greifbar ist, wobei das Dosiseinstellelement zur Einstellung einer aus dem Produktbehälter auszuschüttenden Dosis des Produkts relativ zu dem Gehäuse, insbesondere durch Muskelkraft des Verwenders, verdrehbar oder/und verschiebbar ist und mindestens zwei verschiedene, insbesondere durch Rastpositionen vorgegebene Positionen relativ zu dem Gehäuse einnehmen kann.

Das Einstellen und Verabreichen einer Dosis eines Medikaments insbesondere mit dem Autoinjektor oder Bolusinjektor kann folgende Schritte und entsprechende Relativbewegungen aufweisen:
- Abziehen des Schutzdeckels oder eines Schutzmittels vom Gehäuse mit einer Schraub- oder Zugbewegung
- Abziehen der Nadelhülle von der Spritze mit einer Schraub- oder Zugbewegung
- Einstellen einer zu verabreichenden Dosis durch Bewegen des Dosiseinstellelements
- Betätigen des Knopfs wodurch mit der Auslöseeinrichtung eine Verabreichung entriegelt oder ausgelöst werden kann
- Aufsetzen und Andrücken der Nadelschutzhülse und/oder des Gehäuses auf eine Injektionsstelle wodurch die Nadelschutzhülse in das Gehäuse bewegt wird und mit der Auslöseeinrichtung eine Verabreichung entriegeln oder auslösen kann
- Einstechen der Nadel in die Injektionsstellen durch eine Längsbewegung der Spritze relativ zu der Nadelschutzhülse und/oder des Gehäuses
- Verabreichung des Medikaments wobei die Antriebsfeder dekomprimiert und den Stössel in eine Längsbewegung relativ zu den gehäusefesten Teilen, insbesondere zu der Spritze versetzt wodurch der Stössel mit seinem distalen Ende oder Flansch den Kolben in der Karpule verschiebt, wodurch Medikament durch die Kanüle der Nadel gedrängt wird.
- Entfernen der Nadelschutzhülse von der Injektionsstelle wodurch die Nadelschutzhülse durch die Kraft der Nadelschutzhülsenfeder aus dem Gehäuse bewegt wird bis das distale Ende der Nadelschutzhülse distal über die Nadelspitze der Nadel steht und die Sperreinrichtung durch die Rückstellbewegung die Nadelschutzhülse gegen das Gehäuse verriegelt.
- Aufsetzen des Schutzdeckels oder Schutzmittels auf das Gehäuse

Beispielsweise kann die Verabreichungsvorrichtung auch ein Injektionspen oder Autopen sein, welcher insbesondere folgende Mittel, welche aus einem oder mehreren Geräteteilen gebildet sind, enthalten kann:
- ein Gehäuse, welches vorzugsweise hülsenförmig und länglich mit einer Längsachse gebildet ist und einen vom distalen Ende abziehbaren Schutzdeckel aufweist
- einen Karpulenhalter, welcher mit dem Gehäuse verbindbar ist und eine Karpule aufnimmt welche eine Medikament umschliesst wobei die Karpule einen verschiebbaren Kolben und ein Septum aufweist durch welches eine Kanüle einstechbar ist, welche das Medikament an den Verabreichungsort leitet wobei die Kanüle als Nadel mit dem Karpulenhalter verbindbar ist
- eine Kolbenstange mit Flansch, welcher auf dem Kolben aufliegt. Die Kolbenstange ist versehen mit einem Gewinde und einer Längsführungsnute wobei das Gewinde mit einer Abtriebshülse und die Längsführungsnute mit dem Gehäuse im Eingriff ist oder alternativ das Gewinde mit dem Gehäuse und die Längsführungsnute mit der Abtriebshüse im Eingriff ist
- eine Kupplungshülse welche axial verschiebbar mit der Abtriebshülse gekoppelt ist und über eine Kupplung mit einer Antriebshülse verbindbar ist wobei die Kopplung durch einen Knopf betätigt werden kann
- die Antriebshülse ist mit einem Gewinde versehen welches mit dem Gehäuse im Eingriff bleibt wodurch sich die Antriebshülse entlang der Längsachse relativ zum Gehäuse ein- und ausschrauben lässt oder die Antriebshülse kann gegen oder mit der Kraft eines Federtriebs insbesondere drehend gegen das Gehäuse bewegt werden

Das Einstellen und Verabreichen einer Dosis eines Medikaments insbesondere mit dem Injektionspen oder Autopen kann folgende Schritte und entsprechende Relativbewegungen aufweisen:
- Abziehen des Schutzdeckels vom Gehäuse mit einer Schraub- oder Zugbewegung
- Verbinden einer Nadel mit dem Karpulenhalter mit einer Schraub- oder Druckbewegung
- Einstellen einer zu verabreichenden Dosis durch Schrauben der Antriebshülse im Gehäuse bei nicht betätigtem Knopf
- Vorbereiten der Verabreichung der eingestellten Dosis durch Betätigen des Knopfes wobei insbesondere die Antriebshülse mit der Kupplungshülse gekoppelt wird
- Verabreichung des Medikaments durch Einschrauben bzw. Eindrücken der Antriebshülse oder Drehen derselben mittels des Federtriebs bei betätigtem Knopf wodurch die Kupplungshülse mitgedreht wird welche die Abtriebshülse rotativ mitnimmt welche über den Eingriff mit der Kolbenstange und deren Eingriff ins Gehäuse diese in eine Längsbewegung versetzt wodurch der Flansch den Kolben in der Karpule verschiebt, wodurch Medikament durch die Kanüle gedrängt wird
- Aufsetzen des Schutzdeckels auf das Gehäuse

Das aktivierbare Kennzeichnungsmittel. Erfindungsgemäss umfasst das aktivierbare Kennzeichnungsmittel ein Trägermittel, einen RFID Schaltkreis mit einem Antennenanschluss und zumindest einem Signalanschluss, zumindest einen Aufnehmer welcher mit dem zumindest einen Signalanschluss verbunden ist, sowie eine Antenne welche mit dem Antennenanschluss verbunden ist, wobei das Trägermittel den RFID Schaltkreis, die Antenne und den zumindest einen Aufnehmer aufnimmt und/oder verbindet. Das Trägermittel kann also einen im Wesentlichen zwei- oder dreidimensionalen Aufbau erlauben, ist also bevorzugt als flexible Leiterplatte, Folie oder als 3D-MID "molded interconnected device" Baugruppe aufgebaut. Insbesondere auf seinen flächigen Aussenseiten kann das aktivierbare Kennzeichnungsmittel oder das Trägermittel zumindest einen Aufdruck oder optisch lesbaren Code aufweisen wie bekannt von den hergebrachten Etiketten. Zumindest eine Aussenseite des aktivierbaren Kennzeichnungsmittels kann vermittels eines geeigneten Adhesivs oder aufgrund von verrastbaren, insbesondere formschlüssigen Strukturen auch dazu dienen, das aktivierbare Kennzeichnungsmittel mit Teilen der Verabreichungsvorrichtung fest oder operativ zu verbinden.

Die Auslesevorrichtungen. Erfindungsgemäss erlaubt eine Auslesevorrichtung - wie hinlänglich aus dem Stand der Technik bekannt - das Lesen von zumindest zwei unterschiedlichen Codes aus dem RFID Schaltkreis. Die Auslesevorrichtung versorgt also das aktivierbare Kennzeichnungsmittel vorübergehend mit Energie, dekodiert die gelesene Information und bringt diese direkt oder durch eine App zur Anzeige, speichert die Information beispielsweise und/oder verknüpft die Information mit einem Zeit- oder Datumstempel und/oder überträgt entsprechende Information an übergeordnete Systeme, wie Workstation, Hostcomputer, Netzwerkknoten oder Netzwerkspeicher, insbesondere Cloudspeicher

Die RFID Schaltung, sofern sie von aussen mit Energie versorgt wird, erfasst vom Aufnehmer zumindest ein Signal und codiert und überträgt dieses an eine Auslesevorrichtung . Eine geeignete Antenne erlaubt die elektromagnetische oder induktive Kopplung mit der Auslesevorichtung wodurch Energie und Information übertragbar wird. Verschiedene technische Lösungen und Übertragungsprotokolle, beispielsweise "Nahfeldkommunikation NFC" sind aus dem teilweise zur Norm erhobenen Stand der Technik bekannt.

Die Aufnehmer und deren Signale. Als Aufnehmer kommen passive oder aktive Sensoren zum Einsatz. Passive Sensoren sind insbesondere: Schaltkontakte, trennbare Leiter oder Leiterschleifen, resistive Dehnmessstreifen DMS, variable Widerstände, Kondensatoren oder Potentiometer, galvanisch oder kapazitiv abtastbare Leiterstrukturen für binäre Codes, insbesondere Graycodes, Reedkontakte, Resonanzkreise, Wiegandsensoren. Aktive Sensoren sind insbesondere: Fotoelemente, Fototransistoren, Hallelemente, magnetoresistive Elemente, Piezoelemente, induktive und kapazitive Taster, Thermoelemente, integrierte Schaltungen, Messwandler. Oft sind aktive Sensoren zweiteilig aufgebaut und enthalten Verstärker, wobei ein Sender- oder Feldgeber bzw. Feldbeeinflusser als Geber mit einem entsprechenden Empfänger oder Detektor zusammenwirkt. Entsprechend sind die Signale repräsentiert als zumindest Schalterzustände Ein/Aus, als Analogwert z.B. Widerstand, Spannung oder Stromstärke

Benutzungszustände, Codes und Information. Die genannten Signale sind einem spezifischen Benutzungszustand zugeordnet und bei einer erfindungsgemässen Verabreichungsvorrichtung repräsentiert als zumindest zwei unterscheidbare relative Positionen von einem oder mehreren der Geräteteile welche die Verabreichungsvorrichtung bilden. Auch können absolute Positionen, wie beispielsweise die Lage in einem Gravitationsfeld oder in einem äusseren Magnetfeld bzw. elektromagnetischen Feld oder die Beschleunigungen träger Massen Signale begründen. Die Signale werden in der RFID Schaltung als Codes aufbereitet, welche diese Benutzungszustände charakterisieren. Unmittelbar beim Auslesen oder zeit- oder ortsversetzt dienen diese Codes der Übertragung der Information und/oder zur Synthetisierung von komplexeren Botschaften, welche für einen Benutzer ausgegeben oder gespeichert werden können.

Erfindungsgemässe Anwendungsfälle und Verfahren können folgende Schritte enthalten:
- Lesen eines erstes Codes vom RFID Schaltkreis, wenn sich zumindest ein Geräteteil in einem ersten Zustand oder Position befindet
- Lesen eines zweiten vom ersten unterscheidbaren Codes vom RFID Schaltkreis, wenn sich zumindest ein Geräteteil in einem zweiten Zustand oder Position befindet
- Ausgeben von Botschaften abhängig vom gelesenen Code insbesondere als Text oder Bild auf einer Anzeige oder als Video- oder Audiobotschaft oder URL.
- Verknüpfen der Codes oder Botschaften mit einem Zeitstempel
- Speichern der Codes oder Botschaften in der Auslesevorrichtung oder einem Netzwerkspeicherort, Datenbank oder Cloud-Speicher
- Vergleichen der Codes oder Botschaften mit Vorgabewerten und Ausgeben entsprechender Bestätigungen oder Warnungen
- Übermitteln der Codes oder Botschaften an ein Hostsystem oder ein Netzwerk oder eine Datenbank oder ein Fernsteuergerät oder ein Blutzuckermessgerät oder ein Telemedizinsystem
- Abfragen von Datenbanken und Ausgeben entsprechender Antworten

Systeme und Schnittstellen. Ein erstes System ist aus einer Verabreichungsvorrichtung mit aktivierbarem Kennzeichnungsmittel bildbar, wobei eine erste Schnittstelle die Bedienungsfunktionen für den Benutzer, medizinisches Personal oder logistische Funktionen in Produktion oder Apotheke direkt an der Verabreichungsvorrichtung bereitstellt. Beispielhaft kann dies sein: Abziehen einer Schutzeinrichtung, Auslösen der Injektion, sicher Verschliessen nach Gebrauch.

Ein zweites System wird erfindungsgemäss durch Hinzufügen mindestens einer Auslesevorrichtung zum ersten System gebildet wobei eine zweite Schnittstelle die weiter oben beschriebene RFID - Übertragung und eine dritte Schnittstelle die Bedienungsfunktionen auf der Auslesevorrichtung für den Benutzer, medizinisches Personal oder logistische Funktionen in Produktion oder Apotheke bereitstellt.

Ein drittes System wird durch Hinzufügen mindestens einer netzwerkintegrierten Einheit oder eines Hostrechners zum zweiten System gebildet wobei eine vierte Schnittstelle Netzwerkfunktionen, Client-Server-Transaktionen oder Clouddienste für den Benutzer, medizinisches Personal oder logistische Funktionen in Produktion oder Apotheke oder für Leistungsträger wie beispielsweise Versicherungen bereitstellt.

Der Begriff "Medikament" oder "Produkt" umfasst hier jede medizinische Formulierung, welche geeignet ist zur kontrollierten Verabreichung durch ein Mittel, wie z. B. eine Kanüle oder Hohlnadel oder Düse, hindurch, beispielsweise umfassend eine Flüssigkeit, eine Lösung, ein Gel oder eine feine Suspension oder Aerosol welche(s) einen oder mehrere medizinische Wirkstoffe enthält. "Medikament" kann eine Zusammensetzung mit einem einzigen Wirkstoff oder eine vorgemischte oder co-formulierte Zusammensetzung mit mehreren Wirkstoffen aus einem einzelnen Behälter sein. Medikament umfasst Arzneien wie Peptide (z.B. Insuline, Insulin enthaltende Medikamente, GLP-1 enthaltende sowie abgeleitete oder analoge Zubereitungen), Proteine und Hormone, biologisch gewonnene oder aktive Wirkstoffe, Wirkstoffe auf Basis von Hormonen oder Genen, Nährformulierungen, Enzyme und weitere Substanzen sowohl in fester (suspendierter) oder flüssiger Form aber auch Polysaccharide, Vaccine, DNS oder RNS oder Oglionukleotide, Antikörper oder Teile von Antikörpern sowie geeignete Basis-, Hilfs- und Trägerstoffe.

Substanzen sowohl in fester (suspendierter) oder flüssiger Form aber auch Polysaccharide, Vaccine, DNS oder RNS oder Oglionukleotide, Antikörper oder Teile von Antikörpern sowie geeignete Basis-, Hilfs- und Trägerstoffe.

### Aspekte der Erfindung

Eine Verabreichungsvorrichtung zur parenteralen Verabreichung eines Medikaments aufweisend ein erstes Geräteteil (2, 55, 102, 120)
zumindest ein zweites Geräteteil (3, 61, 103,130) welches vor oder während oder nach einer Verabreichung relativ zum ersten Geräteteil von einer ersten Zustand in zumindest einen zweiten Zustand bewegbar ist,
sowie ein aktivierbares Kennzeichnungsmittel (100) aufweisend
ein Trägermittel (108),
einen RFID Schaltkreis (106) mit einem Antennenanschluss (106b) und zumindest einem Signalanschluss (106a),
zumindest einen Aufnehmer (104,105) welcher zumindest zwei operativ verbundene Aufnehmerteile (104, 105) aufweist von denen zumindest ein Aufnehmerteil (105) mit dem zumindest einen Signalanschluss (106a) verbunden ist,
sowie eine Antenne (107) welche mit dem Antennenanschluss (106b) verbunden ist,
wobei das Trägermittel (108) den RFID Schaltkreis (106), die Antenne (107) und das zumindest eine Aufnehmerteil (105) aufnimmt oder verbindet,
wobei zumindest ein Bereich oder Abschnitt des Trägermittels (108) mit zumindest dem ersten Geräteteil (2, 55,102, 120) verbunden ist, und das zweite Sensorteil (104) mit dem zweiten Geräteteil (3, 61, 103,130) verbunden ist,
wobei eine relative Bewegung des zumindest zweiten Geräteteils (3, 61, 103, 130) zu dem ersten Geräteteil (2, 55, 102, 120) von der ersten in die zumindest zweite Position eine Signaländerung des Sensors (104,105) bewirkt,
wobei die Verabreichungsvorrichtung eine aus Autoinjektor, Injektionspen, Autopen, Infusionpumpe, Patchpumpe, Inhaler oder Bolusinjektor umfasst und zumindest eines aus erstem und zweitem Geräteteil eines aus Nadelschutzhülse (3, 103, 130), Kolben, Flansch (54), Kolbenstange (7, 58), Stössel (7), Dosierhülse (60), Dosierring (61) Antriebselement, Antriebsfeder, Triebfeder, Skalenelement (60), Zählwerk (65), Verriegelungselement (8,15), Antriebsfeder (9), Antriebsmotor, Ratsche (67), Klickelement (66, 67), Rasterarm, Sperrelement (67), Auslöseelement (3, 103, 139, 15,64), Drücker (64), Kupplung (62), Getriebe (58, 65) umfasst.
   bevorzugt weitergebildet, wobei

- der zumindest eine Bereich des Trägermittels (108) auch das zweite Aufnehmerteil (104) aufnimmt und so mit dem zweiten Geräteteil (3, 61, 103,130) verbunden ist, dass eine relative Bewegung des zumindest zweiten Geräteteils (3, 61, 103, 130) zu dem ersten Geräteteil (2, 55,102, 120) von dem ersten in den zumindest zweiten Zustand den zumindest einen Bereich des Trägermittels mechanisch verformt oder trennt, dass die Verformung oder Trennung (109) des zumindest einen Bereichs des Trägermittels (108) die operative Verbindung der zumindest zwei Aufnehmerteile (104, 105) beeinflusst und eine Signaländerung des zumindest einen Aufnehmers (104,105) bewirkt.
- der zumindest eine Aufnehmer (104, 105) zur Signaländerung einen trennbaren Leiter (104) oder einen variablen Widerstand (110) oder einen piezoelektrischen Wandler (110) aufweist.
- die operative Verbindung zwischen dem zumindest einen Aufnehmerteil (105) dem zweiten-Aufnehmerteil (104) galvanisch oder über ein magnetisches oder elektrisches oder elektromagnetisches Feld herstellbar ist dadurch gekennzeichnet, dass der zumindest eine Aufnehmer (104,105) je entsprechende Kontakte (113) und Gegenkontakte (114, 115) beziehungsweise je entsprechende Detektoren (111) und Geber (112) aufweist.
- eine relative Bewegung des zumindest zweiten Geräteteils (3, 61, 103, 130) zu dem ersten Geräteteil (2, 55, 102, 120) von dem ersten in den zumindest zweiten Zustand eine Signaländerung des Aufnehmers (104, 105, 111, 112, 113,114, 115) bewirkt.
- das erste und das zweite Geräteteil je eines aus Gehäuse (2, 55, 102, 120), Schutzdeckel (4, 51), Schutzmittel, Designschale, Griffschale, Mechanikhalter (5, 56), Verpackung, Hülle, Blisterfolie, Nadeladapter, Nadel, Hohlkanüle, Nadelhülle, Karpulen- oder Spritzenhalter (1, 52), Kappe (12), Nadelschutzhülse (3, 103, 130), Abzieher (4), Karpule (53), Septum, Spritze, Kolben, Flansch (54), Kolbenstange (7, 58), Stössel (7), Dosierhülse (60), Dosierring (61) Antriebselement, Antriebsfeder, Triebfeder, Skalenelement (60), Zählwerk (65), Verriegelungselement (8,15), Antriebsfeder (9), Antriebsmotor, Ratsche (67), Klickelement (66, 67), Rasterarm, Sperrelement (67), Haltearm, Führung, Auslöseelement (3, 103, 139, 15,64), Drücker (64), Kupplung (62), Taste, Gewinde (58), Getriebe (58, 65), Anschlag umfasst.
- das Trägermittel flächig ausgebildet ist, lesbare Informationen tragen kann und zumindest teilweise auf der äusseren oder von aussen einsehbaren Oberfläche der Verabreichungsvorrichtung angebracht ist.
- das Trägermittel form- oder kraftschlüssig mit zumindest dem ersten Geräteteil und zumindest teilweise im Innern der Verabreichungsvorrichtung angebracht ist, insbesondere in einem Ringspalt welcher zwischen den Geräteteilen ausgebildet ist.
- der RFID Schaltkreis einer Auslesevorrichtung, insbesondere einem Smartphone, unterscheidbare Codes bereitstellt, welche eine Funktion des Signals sind, welches an dem zumindest einen Signalanschluss anliegt.
- der RFID Schaltkreis kompatibel ist mit Geräten und Systemen welche nach dem Übertragungsprotokoll Nahfeldkommunikation NFC arbeiten.

Weiter ein Verfahren durchgeführt mit einer erfindungsgemässsen Verabreichungsvorrichtung und mindestens einer Auslesevorrichtung, enthaltend mindestens einen der folgende Schritte:
- Lesen eines erstes Codes vom RFID Schaltkreis, wenn sich das zweite Geräteteil in dem ersten Zustand befindet
- Lesen eines zweiten vom ersten unterscheidbaren Codes vom RFID Schaltkreis, wenn sich das zweite Geräteteil in dem zweiten Zustand befindet
weiter enthaltend mindestens einen der folgenden Schritte:
- Ausgeben von Botschaften abhängig vom gelesenen Code insbesondere als Text oder Bild auf einer Anzeige oder als Video- oder Audiobotschaft oder URL.
- Verknüpfen der Codes oder Botschaften mit einem Zeitstempel
- Speichern der Codes oder Botschaften in der Auslesevorrichtung oder einem Netzwerkspeicherort, Datenbank oder Cloud-Speicher
- Vergleichen der Codes oder Botschaften mit Vorgabewerten und Ausgeben entsprechender Bestätigungen oder Warnungen
- Übermitteln der Codes oder Botschaften an ein Hostsystem oder ein Netzwerk oder eine Datenbank oder ein Fernsteuergerät oder ein Blutzuckermessgerät oder ein Telemedizinsystem
- Abfragen von Datenbanken und Ausgeben entsprechender Antworten

### Kurze Beschreibung der Zeichnungen

Figur 1 illustriert am Beispiel eines Autoinjektors beispielhaft die Geräteteile an welchen die Erfindung mit einem aktivierbaren Kennzeichnungsmittel ausgeführt werden kann
Figur 2 illustriert am Beispiel eines Peninjektors beispielhaft die Geräteteile an welchen die Erfindung mit einem aktivierbaren Kennzeichnungsmittel ausgeführt werden kann
Figur 3 zeigt eine erfindungsgemässe Verabreichungsvorrichtung in verschiedenen Benutzungszuständen
Figur 4 zeigt eine erfindungsgemässe Abwandlung des Aufnehmers in einem ersten und zweiten Zustand
Figur 5 zeigt eine erfindungsgemässe Abwandlung des aktivierbaren Kennzeichnungsmittels in einem ersten und zweiten Zustand
Figur 6 zeigt eine weitere erfindungsgemässe Abwandlung des aktivierbaren Kennzeichnungsmittels in einem ersten und zweiten Zustand
Figur 7 zeigt eine weitere erfindungsgemässe Abwandlung des aktivierbaren Kennzeichnungsmittels in einem ersten und zweiten Zustand
Figur 8 zeigt eine weitere erfindungsgemässe Verabreichungsvorrichtung in verschiedenen Benutzungszuständen

### Detailbeschreibung von bevorzugten Ausführungsformen

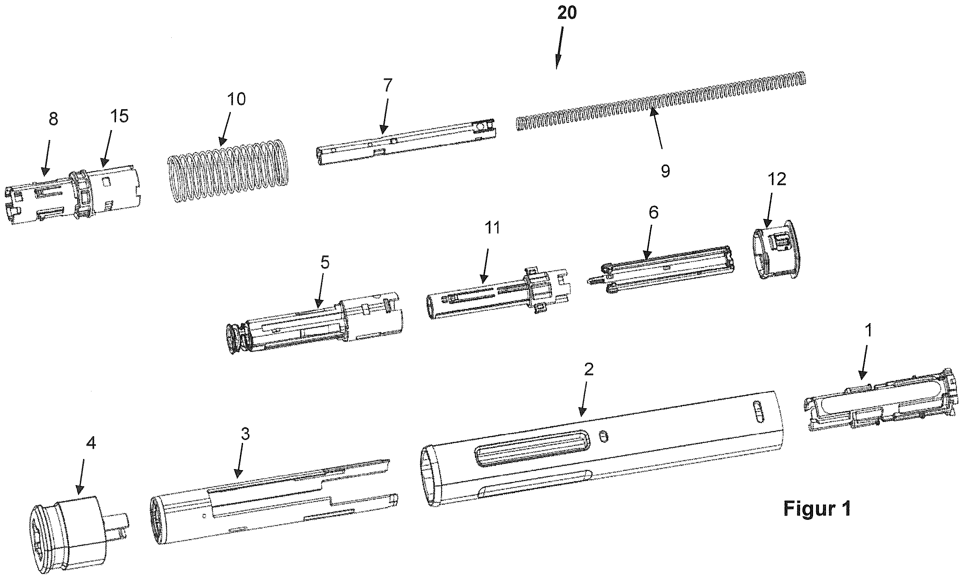

Figur 1 illustriert am Beispiel eines Autoinjektors beispielhaft die Geräteteile an welchen die Erfindung mit einem aktivierbaren Kennzeichnungsmittel ausgeführt werden kann. Grundsätzlich ist die Erfindung an allen Geräteteilen ausführbar, welche insbesondere vor, während oder nach Gebrauch des Autoinjektors oder einer vergleichbaren Verabreichungsvorrichtung unterscheidbare Positionen einnehmen und signifikante Zustände charakterisieren. Erfindungsgemäss können bevorzugt jeweils zumindest zwei der im Folgenden beschriebenen Einzelteile des gezeigten Autoinjektors als erstes und zweites Geräteteil zur Anwendung kommen, sofern diese sich relativ zueinander bewegen und dadurch eine Änderung des Benutzungszustandes begründen:
Bevorzugt zum Gehäuse 2 oder zu gehäusefesten Teilen, insbesondere Spritzenhalter 1, Spritze (nicht gezeigt), Endkappe 12, Mechanikhalter 5 bewegt sich in einer ersten Phase beim Vorbereiten des Autoinjektors für den Gebrauch durch Öffnen, je der Schutzdeckel 4 und gleichzeitig die nadelabdeckende Nadelhülle (nicht gezeigt) welche von der Spritze (nicht gezeigt) entfernt wird.

Anschliessend kann der Autoinjektor auf die Injektionsstelle aufgesetzt und aufgedrückt werden. In dieser zweiten Phase bewegt sich die Nadelschutzhülse 3 zusammen mit der vorderen Sperrhülse 15 und hinteren Sperrhülse 8 und unter Kompression der Nadelschutzfeder 10 bevorzugt zu den erwähnten gehäusefesten Teilen und löst die Verbindung zwischen Stössel 7 und Haltestift 6. Als dritte Phase erfolgt nun die Auschüttung, welche durch eine proximale Verschiebung des Haltestifts 6 mit der hinteren Sperrhülse 8 und Aufschlagen derselben auf den Mechanikhalter 5 eingeleitet wird. Weiter verschiebt sich der nun freigegebene Stössel 7 nach distal wobei er die Klickhülse 11 einen Wegabschnitt mitnimmt wodurch die Nadelschutzfeder 10 weiter komprimiert wird. Diese distale Verschiebung des Stössels 7 wird durch die sich dabei dekomprimiernde Ausschüttfeder 9 bewirkt und hat eine distale Verschiebung des Kolbens (nicht gezeigt) in der Spritze zur Folge. Am Ende dieser distalen Verschiebung des Stössels 7 bzw. der so bewirkten Verdrängung des Medikaments, gibt der Stössel 7 die Klickhülse 11 wieder frei, wodurch diese aufgrund der Kraft der Nadelschutzfeder nach proximal bewegt wird und an der gehäusefesten Endkappe 12 aufschlägt. In der folgenden vierten Phase wird der Autoinjektor von der Injektionsstelle entfernt, wobei die Nadelschutzhülse 3 zusammen mit der vorderen Sperrhülse 15 von der Nadelschutzfeder 10 nach distal bewegt wird. Die Nadelschutzhülse 3 deckt nun die Nadel an der Spritze (nicht gezeigt) wieder ab und die vordere Sperrhülse 15 verriegelt mit der hinteren Sperrhülse 8 derart, dass das so gebildete "ausgezogene Teleskop" ein erneutes Eindrücken der Nadelschutzhülse 3 in das Gehäuse zuverlässig verhindert.

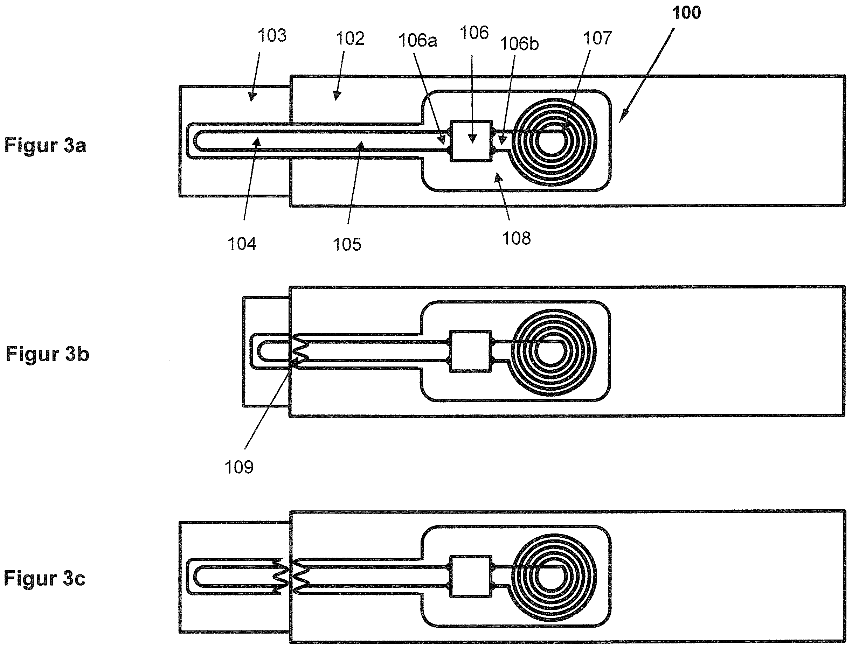

Figuren 3a,b,c zeigen eine erfindungsgemässe Verabreichungsvorrichtung in verschiedenen Benutzungszuständen. Von der Verabreichungsvorrichtung sind vereinfacht und beispielhaft nur das Gehäuse 102 als erstes Geräteteil und die Nadelschutzhülse 103 als zweites Geräteteil gezeigt. Andere Geräteteile, insbesondere wie vorstehend beschrieben sind ebenso geeignet, die Erfindung fortgebildet auszuführen.

Figur 3a zeigt eine erfindungsgemässe Verabreichungsvorrichtung in einem ersten Zustand mit einem aktivierbaren Kennzeichnungsmittel 100 aufweisend das Trägermittel 108 wobei das Trägermittel 108 den RFID Schaltkreis 106, die Antenne 107 und den Aufnehmer 104, 105 mechanisch aufnimmt und auf den Antennenanschluss 106b und den Signalanschluss 106a elektrisch verbindet. Wie gezeigt ist ein Bereich oder Abschnitt 105 mit dem ersten Geräteteil 102 und eine anderer Bereich oder Abschnitt 104 mit dem zweiten Geräteteil 103 operativ verbunden.

Figur 3b zeigt die erfindungsgemässe Verabreichungsvorrichtung in einem zweiten Zustand wobei die Integrität des Aufnehmers 104, 105 durch die relative Bewegung des ersten Geräteteils 102 zum zweiten Geräteteil 103 aufgrund der genannten operativen Verbindung gestört wurde, insbesondere ist in einem dritten Zustand die gezeigte Leiter oder Leiterschleife 104, wie in Figur 3c sichtbar wird, an einer Trennstelle 109 dauerhaft mechanisch und elektrisch unterbrochen.

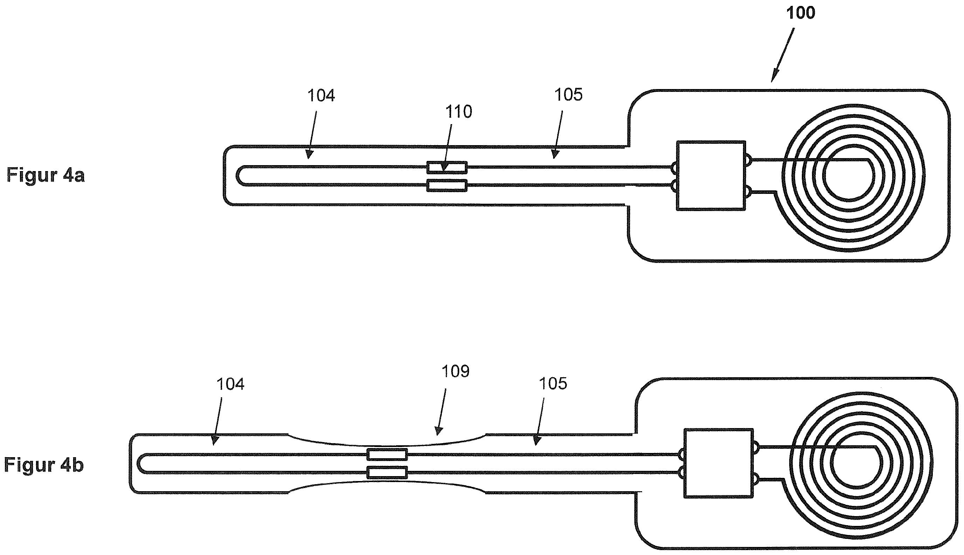

Figuren 4a und 4b zeigen eine erfindungsgemässe Abwandlung des Aufnehmers 104, 105 in einem ersten und zweiten Zustand wobei hier durch die relative Bewegung des ersten Geräteteils 102 zum zweiten Geräteteil 103 aufgrund der genannten operativen Verbindung eine reversible oder irreversible Verformung 109 des Trägermittels und darauf angebrachter elektrischer Elemente 110 erfolgt, wodurch elektrische Parameter, insbesondere Widerstands- oder Impedanzwerte messbar verändert werden.

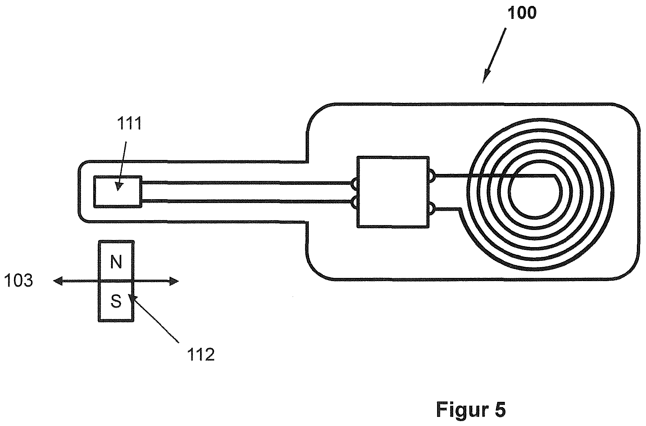

Figur 5 zeigt eine weitere erfindungsgemässe Abwandlung des aktivierbaren Kennzeichnungsmittels 100 wobei hier bevorzugt ein erstes Aufnehmer mit einem Aufnehmer 111 auf dem Trägermittel 108 und mit einem entsprechender Geber 112 auf dem zweiten Geräteteil 103, beispielsweise ein Magnet oder ein ferromagnetisches Element, eine elektrische, induktive oder elektromagnetische Quelle, ein Reflektor, ein Dämpfelement oder einen Lichtleiter bzw. Refraktor aufweist. Wobei die entsprechende Feldkopplung oder operative Verbindung zwischen Aufnehmer 111 und Geber 112 durch die relative Bewegung des ersten Geräteteils 102 zum zweiten Geräteteil 103 messbar verändert wird.

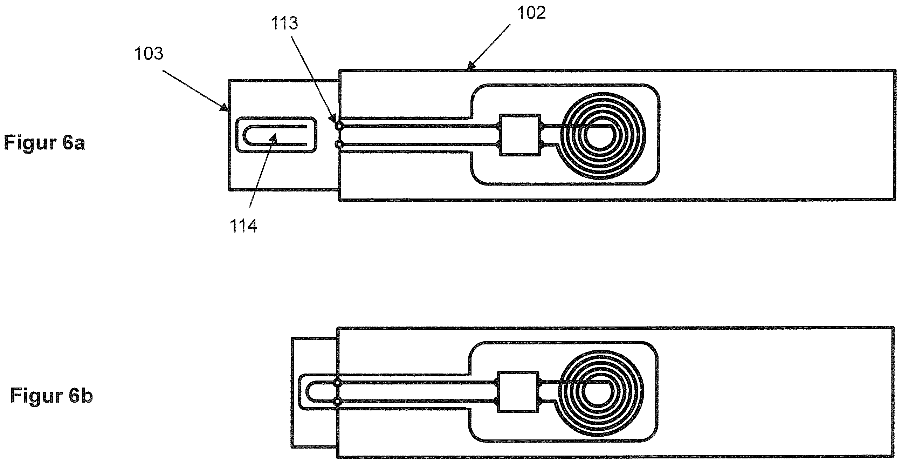

Figuren 6a und 6b zeigen eine weitere erfindungsgemässe Abwandlung des aktivierbaren Kennzeichnungsmittels in einem ersten und zweiten Zustand wobei hier bevorzugt eine galvanische Verbindung mittels Kontakten 113 auf entsprechende Gegenkontakte, vorzugsweise auf einen Leiter 114 reversibel geschlossen oder geöffnet werden in Funktion der relativen Bewegung eines ersten Geräteteils 102 zum zweiten Geräteteil 103.

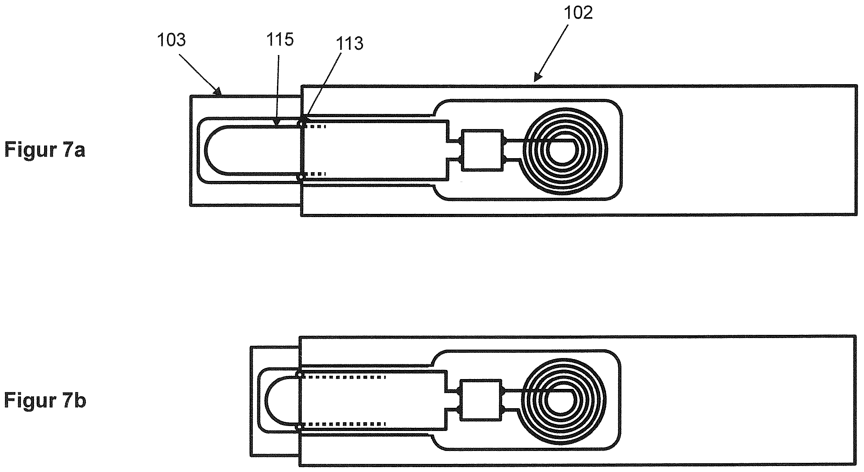

Figuren 7a und 7b zeigen eine weitere erfindungsgemässe Abwandlung des aktivierbaren Kennzeichnungsmittels in einem ersten und zweiten Zustand wobei hier bevorzugt Kontakte 113 einen Leiter mit einem Widerstandsbelag abgreifen, wodurch sich eine variabler Widerstandswert ergibt, welcher eine Funktion der relativen Position des ersten Geräteteils 102 zum zweiten Geräteteil 103 ist.

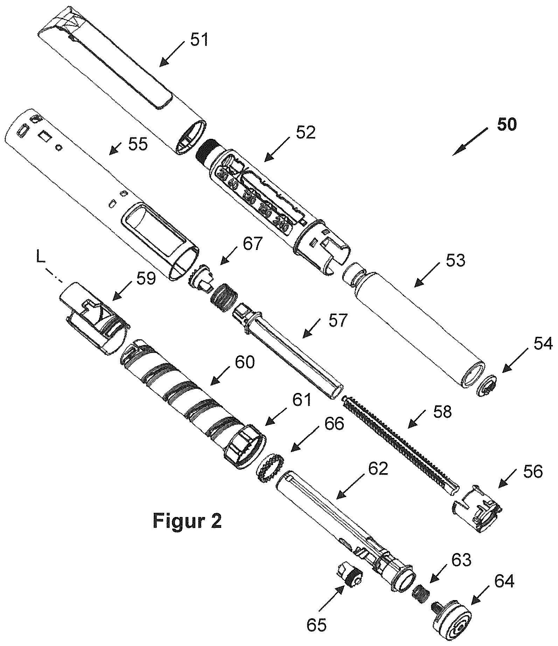

Figur 2 illustriert am Beispiel eines Peninjektors beispielhaft die Geräteteile an welchen die Erfindung mit einem aktivierbaren Kennzeichnungsmittel ausgeführt werden kann. Grundsätzlich ist die Erfindung an allen Geräteteilen ausführbar, welche insbesondere vor, während oder nach Gebrauch des Peninjektors oder einer vergleichbaren Verabreichungsvorrichtung unterscheidbare Positionen einnehmen und signifikante Zustände charakterisieren. Erfindungsgemäss können bevorzugt jeweils zumindest zwei der im Folgenden beschriebenen Einzelteile des gezeigten Autoinjektors als erstes und zweites Geräteteil zur Anwendung kommen, sofern diese sich relativ zueinander bewegen und dadurch eine Änderung des Benutzungszustandes begründen:
Bevorzugt zum Gehäuse 55 mit einer Längsachse L und/oder zu gehäusefesten Teilen, insbesondere Karpulenhalter 52, Karpule 53, Gewindeeinsatz 59, Mechanikhalter 56 bewegt sich in einer ersten Phase beim Vorbereiten des Autoinjektors für den Gebrauch durch Öffnen der Schutzdeckel 51. Eine Nadel mit Hohlkanüle (nicht gezeigt) kann auf das distale Ende des Karpulenhalters 52 aufgesetzt werden. Eine zu verabreichende Dosis kann in einer zweiten Phase durch Drehen am Dosierring 61 aufdosiert bzw. korrigiert werden, wodurch sich die Antriebshülse 60 durch den Gewindeeinsatz 59 aus bzw. in das Gehäuse 55 schraubt. Der frei drehbare Knopf 64 ist während dieser zweiten Phase axial nicht betätigt und der Kupplungsring 66 liegt dadurch locker zwischen der Antriebshülse 60 und der Kupplungshülse 62 wodurch die Kupplungshülse 62 nicht mitgedreht wird. In einer dritten Phase wird Medikament ausgeschüttet, indem der Knopf 64 durch Aufbringen einer axialen Kraft in distaler Richtung betätigt wird, wodurch der Kupplungsring 66 beidseitig kraftschlüssig zwischen der Antriebshülse 60 und der Kupplungshülse 62 gepresst wird und sich die Antriebshülse 60 durch den Gewindeeinsatz 59 zurück in das Gehäuse 55 schraubt. Durch den Kraftschluss wird die Kupplungshülse 62 mitgedreht und gleichzeitig entlang der Achse L distal verschoben. Die Kupplungshülse 62 dreht ihrerseits mittels einer Schiebeführung die axial feste Abtriebshülse 57 mit.

Die Kolbenstange 58 wird nach distal bewegt durch einen Längsführungseingriff und einen Gewindeeingriff mit der Abtriebshülse 57 und dem Gehäuse 55, wodurch der Flansch 54 auf den Kolben (nicht gezeigt) der Karpule 53 drückt und Medikament durch die hohle Nadel gedrängt wird.

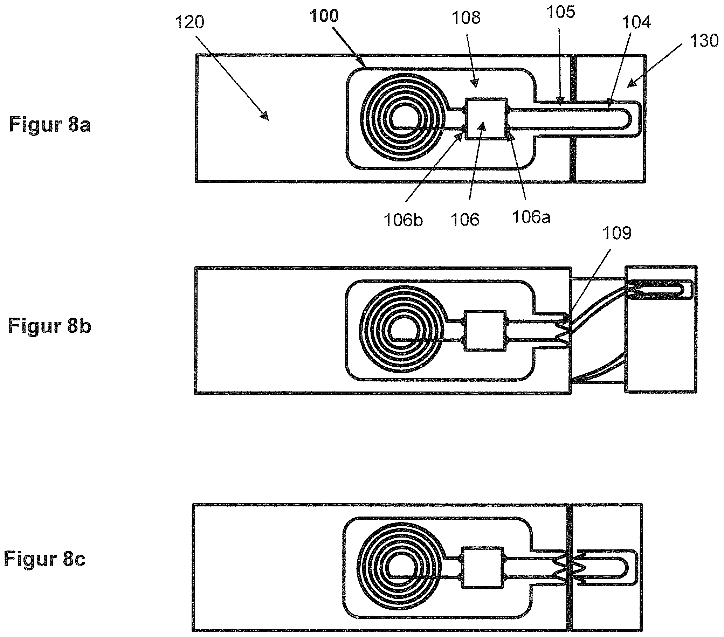

Figur 8a zeigt eine erfindungsgemässe Verabreichungsvorrichtung in einem ersten Zustand mit einem aktivierbaren Kennzeichnungsmittel 100 aufweisend das Trägermittel 108 wobei das Trägermittel 108 den RFID Schaltkreis 106, die Antenne 107 und den Aufnehmer 104, 105 mechanisch aufnimmt und auf den Antennenanschluss 106b und den Signalanschluss 106a elektrisch verbindet. Wie gezeigt ist ein Bereich oder Abschnitt bzw. Aufnehmerteil 105 mit dem ersten Geräteteil 120 und eine anderer Bereich oder Abschnitt bzw. Aufnehmerteil 104 mit dem zweiten Geräteteil 130 operativ verbunden.

Figur 8b zeigt die erfindungsgemässe Verabreichungsvorrichtung in einem zweiten Zustand wobei die Integrität des Trägermittels bzw. des Aufnehmers 104, 105 durch die relative Bewegung des ersten Geräteteils 120 zum zweiten Geräteteil 130 aufgrund der genannten operativen Verbindung gestört wurde, insbesondere ist in einem dritten Zustand die gezeigte Leiter oder Leiterschleife 104, wie in Figur 8c sichtbar wird, an einer Trennstelle 109 dauerhaft mechanisch und elektrisch unterbrochen.

## Patentansprüche

1. Verabreichungsvorrichtung zur parenteralen Verabreichung eines Medikaments aufweisend ein erstes Geräteteil (2, 55, 102, 120)
zumindest ein zweites Geräteteil (3, 61, 103,130) welches während oder nach einer Verabreichung relativ zum ersten Geräteteil von einer ersten Position in zumindest eine zweite Position bewegbar ist,
sowie ein aktivierbares Kennzeichnungsmittel (100) aufweisend
ein Trägermittel (108),
einen RFID Schaltkreis (106) mit einem Antennenanschluss (106b) und zumindest einem Signalanschluss (106a),
zumindest einen aktiven oder passiven Sensor (104,105) welcher zumindest zwei operativ verbundene Sensorteile (104, 105) aufweist von denen zumindest ein Sensorteil (105) mit dem zumindest einen Signalanschluss (106a) verbunden ist,
sowie eine Antenne (107) welche mit dem Antennenanschluss (106b) verbunden ist wobei der RFID Schaltkreis (106) von einer Auslesevorrichtung drahtlos über die Antenne (107) temporär mit Energie versorgt wird und dadurch in der Lage ist Informationen zu einem aktuellen Benutzungszustand der Verabreichungsvorrichtung an die Auslesevorrichtung zu übermitteln und wobei der RFID Schaltkreis (106) der Auslesevorrichtung unterscheidbare Codes bereitstellt, welche eine Funktion eines Signals sind, welches an dem zumindest einen Signalanschluss (106a) anliegt,
wobei das Trägermittel (108) den RFID Schaltkreis (106), die Antenne (107) und das zumindest eine Sensorteil (105) aufnimmt oder verbindet, wobei das Trägermittel flächig ausgebildet ist, lesbare Informationen tragen kann und wobei das Trägermittel form- oder kraftschlüssig mit zumindest dem ersten Geräteteil und zumindest teilweise im Innern der Verabreichungsvorrichtung in einem Ringspalt welcher zwischen den Geräteteilen ausgebildet ist, angebracht ist,
wobei zumindest ein Bereich oder Abschnitt des Trägermittels (108) das zumindest eine Sensorteil (105) aufweist und mit zumindest dem ersten Geräteteil (2, 55,102, 120) verbunden ist und das zweite Sensorteil (104) mit dem zweiten Geräteteil (3, 61, 103, 130) verbunden ist, wobei der zumindest eine Bereich des Trägermittels (108) auch das zweite Sensorteil (104) aufnimmt und so mit dem zweiten Geräteteil (3, 61, 103, 130) verbunden ist, dass eine relative Bewegung des zumindest zweiten Geräteteils (3, 61, 103, 130) zu dem ersten Geräteteil (2, 55, 102, 120) von der ersten in die zumindest zweite Position den zumindest einen Bereich des Trägermittels (108) mechanisch verformt oder trennt,
wobei die relative Bewegung des zumindest zweiten Geräteteils (3, 61, 103, 130) zu dem ersten Geräteteil (2, 55, 102, 120) von der ersten in die zumindest zweite Position eine Signaländerung des Sensors (104, 105) bewirkt
**dadurch gekennzeichnet, dass**
es sich bei der Verabreichungsvorrichtung um eine aus Autoinjektor (20), Injektionspen (50), Autopen, Infusionpumpe, Patchpumpe, Inhaler oder Bolusinjektor handelt und das erste und das zweite Geräteteil je eines aus Gehäuse (2, 55, 102, 120), Schutzdeckel (4, 51), Mechanikhalter (5, 56), Karpulen- oder Spritzenhalter (1, 52), Kappe (12), Nadelschutzhülse (3, 103, 130), Abzieher (4), Karpule (53), Septum, Spritze, Kolben, Flansch (54), Kolbenstange (7, 58), Dosierhülse (60), Dosierring (61), Skalenelement (60), Zählwerk (65), Verriegelungselement (8, 15), Antriebsfeder (9, 10), Antriebsmotor, Ratsche (67), Klickelement (11, 12, 66, 67), Sperrelement (67), Auslöseelement (6, 57), Drücker (64), Kupplung (62), Gewinde (58), Getriebe (58, 65) umfasst.

2. Verabreichungsvorrichtung nach Anspruch 1 **dadurch gekennzeichnet, dass** das Verabreichungsgerät ein Autoinjektor (20) ist, und dass das erste Geräteteil (2, 102) oder zweite Geräteteil (3, 103) eines aus Nadelschutzhülse (3, 103), Kolben, Flansch, Kolbenstange (7), Verriegelungselement (8, 15), Antriebsfeder (9, 10), Antriebsmotor, Klickelement (11, 12), Auslöseelement (6) umfasst.

3. Verabreichungsvorrichtung nach Anspruch 1 oder 2 **dadurch gekennzeichnet, dass** die Verformung oder Trennung (109) des zumindest einen Bereichs des Trägermittels (108) die operative Verbindung der zumindest zwei Sensorteile (104, 105) beeinflusst und eine Signaländerung des zumindest einen Sensors (104,105) bewirkt.

4. Verabreichungsvorrichtung nach Anspruch 3 **dadurch gekennzeichnet, dass** der zumindest eine Sensor (104, 105) zur Signaländerung einen trennbaren Leiter (104) oder einen variablen Widerstand (110) oder einen piezoelektrischen Wandler (110) aufweist.

5. Verabreichungsvorrichtung nach Anspruch 1 oder 2 oder 3 wobei die operative Verbindung zwischen dem zumindest einen Sensorteil (105) dem zweiten Sensorteil (104) galvanisch oder über ein magnetisches oder elektrisches oder elektromagnetisches Feld herstellbar ist **dadurch gekennzeichnet, dass** der zumindest eine Sensor (104, 105) je entsprechende Kontakte (113) und Gegenkontakte (114, 115) beziehungsweise je entsprechende Detektoren (111) und Geber (112) aufweist.

6. Verabreichungsvorrichtung nach einem der vorhergehenden Ansprüche **dadurch gekennzeichnet, dass** das Trägermittel zumindest teilweise auf der äusseren oder von aussen einsehbaren Oberfläche der Verabreichungsvorrichtung angebracht ist.

7. Verabreichungsvorrichtung nach einem der vorhergehenden Ansprüche **dadurch gekennzeichnet, dass** der RFID Schaltkreis (106) kompatibel ist mit Geräten und Systemen welche nach dem Übertragungsprotokoll Nahfeldkommunikation NFC arbeiten.

8. Verfahren durchgeführt mit einer Verabreichungsvorrichtung nach einem der vorhergehenden Ansprüche und mindestens einer Auslesevorrichtung, enthaltend mindestens einen der folgenden Schritte:
- Lesen eines erstes Codes vom RFID Schaltkreis, wenn sich das zweite Geräteteil in der ersten Position befindet
- Lesen eines zweiten vom ersten unterscheidbaren Codes vom RFID Schaltkreis, wenn sich das zweite Geräteteil in der zweiten Position befindet.

9. Verfahren nach Anspruch 8 enthaltend weiter mindestens einen der folgenden Schritte:
- Ausgeben von Botschaften abhängig vom gelesenen Code insbesondere als Text oder Bild auf einer Anzeige oder als Video- oder Audiobotschaft oder URL.
- Verknüpfen der Codes oder Botschaften mit einem Zeitstempel
- Speichern der Codes oder Botschaften in der Auslesevorrichtung oder einem Netzwerkspeicherort, Datenbank oder Cloud-Speicher
- Vergleichen der Codes oder Botschaften mit Vorgabewerten und Ausgeben entsprechender Bestätigungen oder Warnungen
- übermitteln der Codes oder Botschaften an ein Hostsystem oder ein Netzwerk oder eine Datenbank oder ein Fernsteuergerät oder ein Blutzuckermessgerät oder ein Telemedizinsystem
- Abfragen von Datenbanken und Ausgeben entsprechender Antworten

## Claims

1. An administration device for parenteral administration of a medicament having a first device part (2, 55, 102, 120)
at least a second device part (3, 61, 103, 130) which is moveable from a first position into at least a second position relative to the first device part during or after an administration,
and an activatable tagging means (100) comprising
a carrier means (108),
an RFID circuit (106) having an antenna terminal (106b) and at least one signal terminal (106a),
at least one active or passive sensor (104, 105) which has at least two operatively connected sensor parts (104, 105) of which at least one sensor part (105) is connected to the at least one signal terminal (106a),
and an antenna (107) which is connected to the antenna terminal (106b), wherein the RFID circuit (106) is temporarily and wirelessly via the antenna (107) provided with energy from a read-out device, such as to be able to transmit information concerning an operating status of the administration device to the read-out device, and wherein RFID circuit (106) provides distinguishable codes to the read-out device, said codes being a function of the signal which is applied at the at least one signal terminal,
wherein the carrier means (108) receives or connects the RFID circuit (106), the antenna (107) and the at least one sensor part (105), formed flat, can carry readable information, and wherein the carrier means is positively or non-positively attached to at least the first device part and at least partially in the interior of the administration device, in particular in an annular gap which is embodied between the device parts,
wherein at least one region or section of the carrier means (108) comprises the at least one sensor part (105) and is connected to at least the first device part (2, 55, 102, 120) and the second sensor part (104) is connected to the second device part (3, 61, 103, 130), wherein the at least one region of the carrier means (108) also receives the second sensor part (104) and is thus connected to the second device part (3, 61, 103, 130), such that the relative movement of the at least second device part (3, 61, 103, 130) to the first device part (2, 55, 102, 120) from the first to the at least second position mechanically deforms or separates the at least one region of the carrier means,
wherein the relative movement of the at least second device part (3, 61, 103, 130) to the first device part (2, 55, 102, 120) from the first to the at least second position causes a signal change of the sensor (104, 105)
**characterized in that**
the administration device is one of an autoinjector (20), injection pen (50), autopen, infusion pump, patch pump, inhaler or bolus injector, and the first and the second device part each comprise one of a housing (2, 55, 102, 120), protective cover (4, 51), mechanism holder (5, 56), carpule or syringe holder (1, 52), cap (12), protective needle sleeve (3, 103, 130), abductor (4), carpule (53), septum, syringe, piston, flange (54), piston rod (7, 58), dosing sleeve (60), dosing ring (61), scale element (60), counter (65), locking element (8, 15), drive spring (9, 10), drive motor, ratchet (67), click element (11, 12, 66, 67), blocking element (67), trigger element (6, 57), pusher (64), coupling (62), thread (58), gearbox (58, 65).

2. The administration device according to claim 1, **characterized in that** the administration device is an autoinjector (20), and **in that** the first device part (2, 102) or the second device part (3, 103) comprises one of a protective needle sleeve (3, 103), piston, flange, piston rod (7), locking element (8, 15), drive spring (9, 10), drive motor, click element (11, 12), trigger element (6).

3. The administration device according to claim 1 or 2, **characterized in that** the deformation or separation (109) of the at least one region of the carrier means (108) influences the operative connection of the at least two sensor parts (104, 105) and causes a signal change of the at least one sensor (104, 105).

4. The administration device according to claim 3, **characterized in that** the at least one sensor (104, 105) has a separable conductor (104) or a variable resistor (110) or a piezoelectrical transducer (110) for the signal change.

5. The administration device according to claim 1 or 2 or 3, wherein the operative connection between the at least one sensor part (105) [and] the second sensor part (104) can be established galvanically or via a magnetic or electric or electromagnetic field **characterized in that** the at least one sensor (104, 105) has respective corresponding contacts (113) and counter-contacts (114, 115) or respective corresponding detectors (111) and encoders (112).

6. The administration device according to any of the foregoing claims, **characterized in that** the carrier means is at least partially attached to the outer surface or externally visible surface of the administration device.

7. The administration device according to any of the foregoing claims, **characterized in that** the RFID circuit (106) is compatible with devices and systems which work according to NFC near field communication protocol.

8. A method carried out with an administration device according to any of the foregoing claims and at least one read-out device, comprising at least one of the following steps:
- reading a first code from the RFID circuit, when the second device part is in the first position
- reading a second code distinguishable from the first one from the RFID circuit, when the second device part is in the second position.

9. The method according to claim 8, further comprising at least one of the following steps:
- outputting messages depending on the read code in particular as text or image on a display or as a video or audio message or URL,
- linking the codes or messages to a time stamp
- storing the codes or messages in the read-out device or a network memory location, database or cloud storage
- comparing the codes or messages with default values and outputting corresponding confirmations or warnings
- transmitting the codes or messages to a host system or a network or a database or a remote-control device or a blood glucose meter or a telemedicine system
- querying databases and outputting corresponding responses.

## Revendications

1. Dispositif d'administration pour l'administration parentérale d'un médicament présentant une première partie d'appareil (2, 55, 102, 120)
au moins une deuxième partie d'appareil (3, 61, 103, 130), laquelle est mobile pendant ou après une administration par rapport à la première partie d'appareil à partir d'une première position dans au moins une deuxième position,
ainsi qu'un moyen de marquage activable (100) présentant
un moyen de support (108),
un circuit RFID (106) avec une borne d'antenne (106b) et au moins une borne de signal (106a),
au moins un capteur (104, 105) actif ou passif, lequel présente au moins deux parties de capteur (104, 105) reliées de manière opérationnelle dont au moins une partie de capteur (105) est reliée à la au moins une borne de signal (106a),
ainsi qu'une antenne (107), laquelle est reliée à la borne d'antenne (106b), dans lequel le circuit RFID (106) est alimenté temporairement et au moyen de l'antenne (107) d'un dispositif de lecture, pour transmettre des informations sur une condition d' opération actuelle du dispositif d'administration au dispositif de lecture, dans lequel le circuit RFID fournit des codes différenciables, lesquels dépendent du signal, lequel s'applique sur la au moins une borne de signal, au dispositif de lecture,
dans lequel le moyen de support (108) reçoit ou relie le circuit RFID (106), l'antenne (107) et la au moins une partie de capteur (105), dans lequel le moyen de support est réalisé de manière plate, peut porter des informations lisibles, et dans lequel le moyen de support est monté par coopération de formes ou à force avec au moins la première partie d'appareil et au moins en partie à l'intérieur du dispositif d'administration, en particulier dans une fente annulaire, laquelle est réalisée entre les parties d'appareil,
dans lequel au moins une zone ou partie du moyen de support (108) présente la au moins une partie de capteur (105) et est reliée à au moins la première partie d'appareil (2, 55, 102, 120) et la deuxième partie de capteur (104) est reliée à la deuxième partie d'appareil (3, 61, 103, 130), dans lequel la au moins une zone du moyen de support (108) reçoit également la deuxième partie de capteur (104) et est reliée à la deuxième partie d'appareil (3, 61, 103, 130), de sorte que le mouvement relatif de la au moins deuxième partie d'appareil (3, 61, 103, 130) par rapport à la première partie d'appareil (2, 55, 102, 120) à partir de la première dans la au moins deuxième position déforme mécaniquement ou sépare la au moins une zone du moyen de support,
dans lequel le mouvement relatif de la au moins deuxième partie d'appareil (3, 61, 103, 130) par rapport à la première partie d'appareil (2, 55, 102, 120) à partir de la première dans la au moins deuxième position provoque une modification de signal du capteur (104, 105)
**caractérisé en ce que**
le dispositif d'administration est un parmi un auto-injecteur (20), stylo injecteur (50), autopen, pompe à perfusion, pompe patch, inhalateur ou injecteur de bolus, et la première et la deuxième partie d'appareil comprend respectivement un parmi un boîtier (2, 55, 102, 120), un couvercle de protection (4, 51), des supports de mécanisme (5, 56), des porte-carpule ou seringue (1, 52), un capuchon (12), un manchon de protection d'aiguille (3, 103, 130), un extracteur (4), une carpule (53), un septum, une seringue, un piston, une bride (54), une tige de piston (7, 58), un manchon de dosage (60), une bague de dosage (61), un élément gradué (60), un mécanisme compteur (65), un élément de verrouillage (8, 15), un ressort d'entraînement (9, 10), un moteur d'entraînement, un cliquet (67), un élément à déclic (11, 12, 66, 67), un élément de blocage (67), un élément de déclenchement (6, 57), une gâchette (64), un accouplement (62), un filetage (58), une transmission (58, 65).

2. Dispositif d'administration selon la revendication 1, **caractérisé en ce que** le dispositif d'administration est un auto-injecteur (20), et **en ce que** la première partie d'appareil (2, 102) ou la deuxième partie d'appareil (3, 103) est un parmi un manchon de protection d'aiguille (3, 103), un piston, une bride, une tige de piston (7, 58), un élément de verrouillage (8, 15), un ressort d'entraînement (9, 10), un moteur d'entraînement, un élément à déclic (11, 12), un élément de déclenchement (6).

3. Dispositif d'administration selon la revendication 1 ou 2, **caractérisé en ce que** la déformation ou séparation (109) de la au moins une zone du moyen de support (108) influence la liaison opérationnelle des au moins deux parties de capteur (104, 105) et provoque une modification de signal du au moins un capteur (104, 105).

4. Dispositif d'administration selon la revendication 3, **caractérisé en ce que** le au moins un capteur (104, 105) présente pour la modification de signal un conducteur (104) séparable ou une résistance (110) variable ou un convertisseur piézoélectrique (110).

5. Dispositif d'administration selon la revendication 1 ou 2 ou 3, dans lequel la liaison opérationnelle entre la au moins une partie de capteur (105) [et] la deuxième partie de capteur (104) peut être établie de manière galvanique ou par l'intermédiaire d'un champ magnétique ou électrique ou électromagnétique, **caractérisé en ce que** le au moins un capteur (104, 105) présente respectivement des contacts (113) et contacts complémentaires (114, 115) correspondants ou respectivement des détecteurs (111) et transmetteurs (112) correspondants.

6. Dispositif d'administration selon l'une quelconque des revendications précédentes, **caractérisé en ce que** le moyen de support est monté au moins en partie sur la surface extérieure ou visible de l'extérieur du dispositif d'administration.

7. Dispositif d'administration selon l'une quelconque des revendications précédentes, **caractérisé en ce que** le circuit RFID (106) est compatible avec des appareils et systèmes, lesquels fonctionnent selon le protocole de transmission communication en champ proche NFC.

8. Procédé mis en œuvre avec un dispositif d'administration selon l'une quelconque des revendications précédentes et au moins un dispositif de lecture, contenant au moins une des étapes suivantes :
- la lecture d'un premier code provenant du circuit RFID, lorsque la deuxième partie d'appareil se trouve dans la première position
- la lecture d'un deuxième code pouvant être différencié du premier provenant du circuit RFID, lorsque la deuxième partie d'appareil se trouve dans la deuxième position.

9. Procédé selon la revendication 8 contenant en outre au moins une des étapes suivantes :
- la délivrance de messages en fonction du code lu en particulier sous la forme de texte ou d'image sur un écran ou sous la forme d'un message vidéo ou audio ou d'une URL
- la combinaison des codes ou messages avec une estampille temporelle
- la mise en mémoire des codes ou messages dans le dispositif de lecture ou un emplacement réseau, une base de données ou un stockage en nuage
- la comparaison des codes ou messages à des valeurs de consigne et la délivrance de confirmations ou d'avertissements correspondants
- la transmission des codes ou messages à un système hôte ou un réseau ou une base de données ou un appareil de commande à distance ou un glucomètre ou un système de télémédecine
- la consultation de bases de données et la délivrance de réponses correspondantes.
